# EUROPEAN PATENT APPLICATION

(11) **EP 2 181 644 A1**
(43) Date of publication of application: **05.05.2010**
(21) Application number: 09000563.8
(22) Date of filing: 16.01.2009
(51) Int. Cl.: A61B 1/247

(54) **Reflecting-type optical inspection apparatus an related dental inspection system**

(30) Priority: 04.11.2008 US 264575
(71) Applicant: AnMo Electronics Corporation,, Hsinchu City (TW)
(72) Inventor: Wu, Paul Neng-Wei, Hsinchu City (TW)
(74) Representative: Weber, Martin

(57) **Abstract**

A reflecting-type optical inspection apparatus (210, 800, 1100) is disclosed including: a holding portion (320, 326; 820, 826; 1120, 1126) configured for detachably holding an external device (310) having a reflective surface (314); a connecting portion (340, 350, 380; 840, 850, 880; 1150, 1180) engaged with the holding portion (320, 326; 820, 826; 1120, 1126); and a digital image sensing module (390) engaged with the connecting portion (340, 350, 380; 840, 850, 880; 1150, 1180) for sensing light reflected from the reflective surface (314) of the external device (310).

## Description

The present invention relates to a reflecting-type optical inspection apparatus according to the pre-characterizing clause of claims 1, 17 and 18.

A conventional intra-oral inspection system includes an intra-oral camera, a computer, and a display. There is a disadvantage associated with the conventional intra-oral inspection system. In particular, a diagnostician would often have to move the intra-oral camera for different viewing angles. However, when the diagnostician moves the intra-oral camera to observe different sides of an object, the image shown on the display often rotates according to changes in the viewing angle of the camera module. Shifting images on the display are distracting to the viewer. This inconveniences the diagnostician watching the image shown on the display and adversely affects the efficiency of the inspection. In addition, the bulky nature of the intra-oral camera also means that certain areas of the mouth cavity will be visually inaccessible. Moreover, post-use disinfection of the intra-oral camera is troublesome, as the intra-oral camera contains electronic components.

In view of the foregoing, there is a need for an inspection apparatus that can mitigate or obviate the aforementioned issues in the related art.

This in mind, the present invention aims at providing a reflecting-type optical inspection apparatus and system that allow a user to rotate an associated reflective surface as needed while maintaining the image shown on a display more or less in the same orientation and reduce visually inaccessible areas.

This is achieved by a reflecting-type optical inspection apparatus according to claim 1, 17 or 18. The dependent claims pertain to corresponding further development and improvements.

The following aspects relate to preferred embodiments of the invention:
1. A reflecting-type optical inspection apparatus comprising:
   a holding portion comprising:
      a first connector; and
      a holding component configured for detachably holding an external device having a reflective surface;
   a connecting portion comprising:
      a body having a hollow portion; and
      a second connector formed on the body and engaged with the first connector of the holding portion; and
   a digital image sensing module disposed in the hollow portion of the body for sensing light reflected from the reflective surface.
2. The reflecting-type optical inspection apparatus of clause 1, wherein the second connector of the connecting portion is detachably engaged with the first connector of the holding portion, and/or the digital image sensing module is detachably disposed in the hollow portion of the body.
3. The reflecting-type optical inspection apparatus of clause 1, wherein the digital image sensing module comprises:
   a casing having a through hole defined longitudinally through the casing and an opening defined on the casing;
   a cap attached to the casing and having a hollow portion;
   at least one guide disposed between the casing and the cap; and
   a telescopic tube assembly disposed within the casing and comprising
      an outer tube rotatably disposed within the casing and having an inner surface, at least one spiral track formed on the inner surface, and a knob corresponding to the opening of the casing;
      an inner tube movably disposed within the outer tube and having a through hole, at least one engaging element movably contacted with the at least one guide and at least one guiding element movably contacted with the at least one spiral track of the outer tube; and
      a lens attached to the through hole of the inner tube.
4. The reflecting-type optical inspection apparatus of clause 3, wherein
   the at least one engaging element of the inner tube comprises a first through hole; and
   the at least one guide comprises a guiding rod passing through the first through hole.
5. The reflecting-type optical inspection apparatus of clause 3, wherein
   the cap is provided with at least one first limiting structure;
   the casing is provided with at least one second limiting structure; and
   each guide has two ends respectively corresponding to the first limiting structure and the second limiting structure.
6. The reflecting-type optical inspection apparatus of clause 3, wherein the digital image sensing module further comprises:
   at least one light source disposed at one end of the digital image sensing module;
   wherein the electricity to the at least one light source is conducted via the at least one guide.
7. The reflecting-type optical inspection apparatus of clause 1, wherein the digital image sensing module further comprises:
   a photo sensor assembly for processing sensed images in response to a control signal.
8. The reflecting-type optical inspection apparatus of clause 7, wherein the digital image sensing module further comprises:
   a control interface disposed on the digital image sensing module and electrically coupled with the photo sensor assembly for triggering the control signal in response to a user operation.
9. The reflecting-type optical inspection apparatus of clause 1, wherein the holding component comprises at least a flexible sleeve or a clamping device.
10. The reflecting-type optical inspection apparatus of clause 1, wherein the holding portion further comprises:
   a fastener detachably engaged with the holding component.
11. The reflecting-type optical inspection apparatus of clause 10, wherein the first connector is attached to the fastener or the holding component.
12. The reflecting-type optical inspection apparatus of clause 10, wherein the fastener comprises a sleeve, a clamping device, or a hoop.
13. The reflecting-type optical inspection apparatus of clause 1, wherein the body of the connecting portion further comprises:
   an inner portion having the hollow portion; and
   an outer portion rotatably engaged with the inner portion;
   wherein the second connector of the connecting portion is formed on the outer portion.
14. The reflecting-type optical inspection apparatus of clause 13, wherein the body of the connecting portion further comprises:
   at least one limiting structure contacted with the outer portion.
15. The reflecting-type optical inspection apparatus of clause 14, wherein the at least one limiting structure is fixedly attached to the inner portion or the outer portion, formed on the inner portion or the outer portion, or detachable from the inner portion and the outer portion.
16. The reflecting-type optical inspection apparatus of clause 1, wherein the second connector of the connecting portion is configured rotatable with respect to the digital image sensing module.
17. The reflecting-type optical inspection apparatus of clause 16, wherein the body of the connecting portion further comprises:
   an inner portion having the hollow portion; and
   an outer portion rotatably engaged with the inner portion;
   wherein the second connector of the connecting portion is formed on the outer portion.
18. The reflecting-type optical inspection apparatus of clause 16, further comprising:
   one or more positioning devices configured for guiding the rotation angle of the second connector of the connecting portion;
   wherein the one or more positioning devices are disposed on at least one of the holding portion, the connecting portion, and the digital image sensing module.
19. The reflecting-type optical inspection apparatus of clause 18, wherein the one or more positioning devices comprise a cavity and/or a protrusion.
20. A reflecting-type optical inspection apparatus comprising:
   a holding component configured for detachably holding an external device having a reflective surface;
   a fastener detachably engaged with the holding component;
   a first connector attached to the fastener or the holding component;
   an inner portion having a hollow portion;
   an outer portion rotatably engaged with the inner portion;
   at least one limiting structure fixedly attached to the inner portion or the outer portion, formed on the inner portion or the outer portion, or detachable from the inner portion and the outer portion;
   one or more positioning devices configured for positioning the holding component to a predetermined position;
   a second connector formed on the outer portion and detachably engaged with the first connector; and
   a digital image sensing module detachably disposed in the hollow portion of the inner portion for sensing light reflected from the reflective surface.
21. A reflecting-type optical inspection apparatus comprising:
   a holding portion configured for detachably holding an external device having a reflective surface;
   a connecting portion engaged with the holding portion; and
   a digital image sensing module engaged with the connecting portion for sensing light reflected from the reflective surface.
22. The reflecting-type optical inspection apparatus of clause 21, wherein the digital image sensing module comprises:
   a casing having a through hole defined longitudinally through the casing and an opening defined on the casing;
   a cap attached to the casing and having a hollow portion;
   at least one guide disposed between the casing and the cap;
   an outer tube rotatably disposed within the casing and having an inner surface, at least one spiral track formed on the inner surface, and a knob corresponding to the opening of the casing;
   an inner tube movably disposed within the outer tube and having a through hole, at least one engaging element movably contacted with the at least one guide and at least one guiding element movably contacted with the at least one spiral track of the outer tube; and
   a lens attached to the through hole of the inner tube.
23. A dental inspection system comprising:
   a dental mirror having a handle and a reflective surface;
   a holding portion detachably engaged with the handle of the dental mirror;
   a handheld digital image sensing module for sensing light reflected from the reflective surface;
   a connecting portion engaged with the holding portion and the handheld digital image sensing module; and
   a display electrically coupled with the handheld digital image sensing module for displaying images sensed by the handheld digital image sensing module;
wherein the holding portion or at least a part of the connecting portion is rotatable with respect to the handheld digital image sensing module.

All combinations and sub-combinations of the above-described features also belong to the invention.

As will be seen more clearly from the detailed description following below, the claimed reflecting-type optical inspection apparatus includes a holding portion for detachably holding an external device having a reflective surface and utilizes a digital image sensing module to obtain images of an object through light reflected from the reflective surface.

In the following, the invention is further illustrated by way of example, taking reference to the accompanying drawings. Thereof
FIG. 1 is a schematic diagram of an intra-oral inspection system according to the related art;
FIG. 2 is a schematic diagram of a reflecting-type optical inspection system according to an exemplary embodiment;
FIG. 3 is a perspective view of the reflecting-type optical inspection apparatus depicted in FIG. 2 according to a first exemplary embodiment;
FIG. 4 is an exploded view of the reflecting-type optical inspection apparatus of FIG. 3;
FIG. 5 is a partial exploded view of the reflecting-type optical inspection apparatus of FIG. 3;
FIG. 6 is another perspective view of the reflecting-type optical inspection apparatus of FIG. 3;
FIG. 7 is an exploded view of the handheld digital image sensing module depicted in FIG. 3, according to an exemplary embodiment;
FIG. 8 is a perspective view of a reflecting-type optical inspection apparatus, according to a second exemplary embodiment;
FIG. 9 is an exploded view of the reflecting-type optical inspection apparatus of FIG. 8;
FIG. 10 is a partial exploded view of the reflecting-type optical inspection apparatus of FIG. 8;
FIG. 11 is a perspective view of a reflecting-type optical inspection apparatus according to a third exemplary embodiment;
FIG. 12 is an exploded view of the reflecting-type optical inspection apparatus of FIG. 11; and
FIG. 13 is a partial exploded view of the reflecting-type optical inspection apparatus of FIG. 11.

Mouth mirrors are widely used to assist viewing while performing work in a patient's oral cavity so that a diagnostician, such as a dentist or an ear-nose-and-throat doctor, may, via the reflection of the mouth mirror, obtain an indirect view of otherwise visually-inaccessible areas of the mouth. Also, mouth mirrors could be used to retract the tongue or cheek in order to view an area directly.

A conventional mouth mirror has a handle and a reflective mirror. The handle is longitudinal and has a front end. The reflective mirror is mounted on the front end of the handle and has a mirror surface. When the conventional mouth mirror is used, the reflective mirror is inserted into a patient's mouth cavity to reflect oral tissue. The size of the reflective mirror is as small as a coin for easy placement into the mouth cavity. Due to the limited size of the reflective mirror, however, a diagnostician often has to strain for a good view. Furthermore, the patient does not have a view of his/her own mouth tissue through the mouth mirror, so he or she may not be aware of the condition inside the mouth cavity.

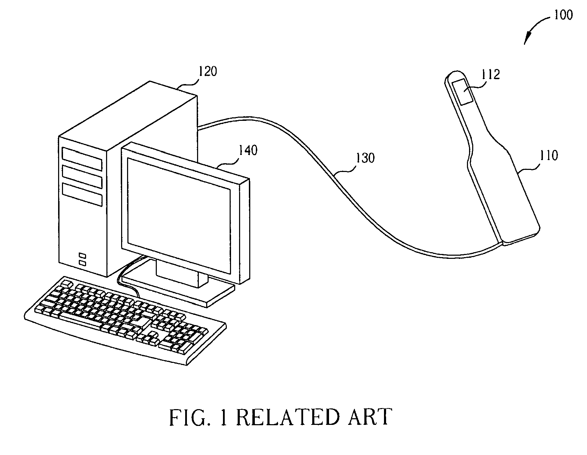

Fig. 1 shows an intra-oral inspection system 100 according to the related art. The intra-oral inspection system 100 includes an intra-oral camera 110, a computer 120 connected to the intra-oral camera 110 via a cable 130, and a display 140. As shown in Fig. 1, the intra-oral camera 110 has a handle, a head portion, and a camera module 112 situated in the head portion. To observe oral tissue, a diagnostician would insert the head portion and part of the handle of the intra-oral camera 110 into a patient's mouth cavity. The images obtained by camera module 112 would be transmitted to the computer 120 via the cable 130 and displayed on the display 140.

Reference will now be made in detail to exemplary embodiments of the invention, which are illustrated in the accompanying drawings. The same reference numbers may be used throughout the drawings to refer to the same or like parts.

Certain terms are used throughout the description and following claims to refer to particular components. As one skilled in the art will appreciate, manufacturers may refer to a component by different names. This document does not intend to distinguish between components that differ in name but not in function. In the following description and in the claims, the terms "include" and "comprise" are used in an open-ended fashion, and thus should be interpreted to mean "include, but not limited to ...".

In addition, some embodiments may be described in the context of dentistry; however, such descriptions are exemplary only and shall in no way be interpreted to limit the use of the present invention to the dental field. The reflecting-type optical inspection apparatus and related system of the present invention have practical applications in many fields including, but not limited to, automotive applications, medicine-related applications, industrial applications, and personal hobby applications.

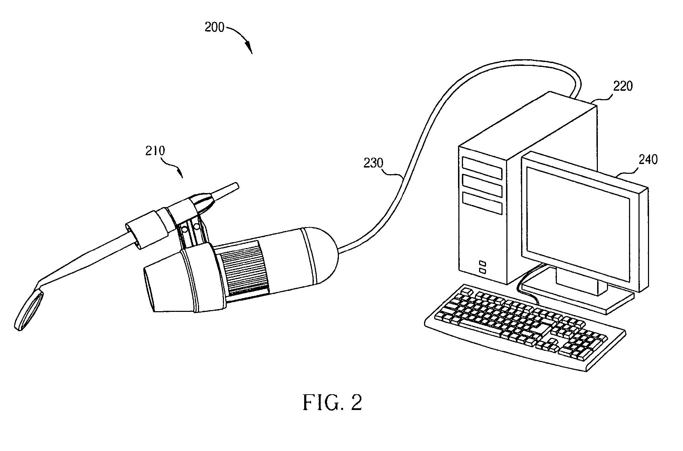

FIG. 2 illustrates a schematic diagram of a reflecting-type optical inspection system 200, according to an exemplary embodiment. The reflecting-type optical inspection system 200 comprises a reflecting-type optical inspection apparatus 210, a computer 220 coupled with the reflecting-type optical inspection apparatus 210 via a connection 230, and a display 240 coupled with the computer 220, wherein the connection 230 may be a physical cable or a wireless link. The term "couple" used herein is intended to mean any indirect or direct connection (e.g., an communication connection; not limited to electrical or optical connections). Accordingly, the display 240 is also coupled with the reflecting-type optical inspection apparatus 210. In applications, the computer 220 and the display 240 may be replaced by a television or other monitoring devices. For the purpose of explanatory convenience in the following description, it is assumed herein that the reflecting-type optical inspection system 200 is a dental inspection system.

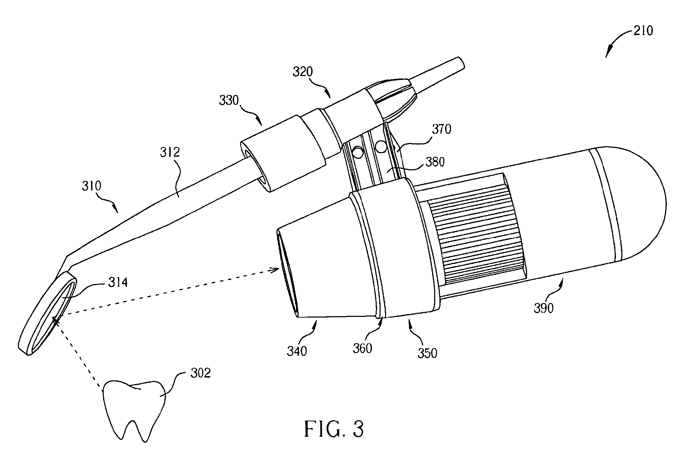

FIG. 3 illustrates a perspective view of the reflecting-type optical inspection apparatus 210 depicted in FIG. 2, according to a first exemplary embodiment. The reflecting-type optical inspection apparatus 210 comprises a holding component 320, a fastener 330, an inner portion 340, an outer portion 350, a limiting structure 360, and a handheld digital image sensing module 390. The holding component 320 is configured for detachably holding an external device (e.g., a dental mirror in this embodiment) 310 having a reflective surface 314. The fastener 330 is detachably engaged with the holding component 320. The outer portion 350 is rotatably engaged with the inner portion 340 and detachably engaged with the holding component 320. The limiting structure 360 is detachably attached to the outer portion 350 and further secures the outer portion 350 onto the inner portion 340. The handheld digital image sensing module 390 is detachably engaged with the inner portion 340. In operations, the handheld digital image sensing module 390 senses light reflected from the reflective surface 314 to obtain images of a target object (such as a tooth) 302, and transmits obtained images to the computer 220 via the connection 230 so as to show the images on the display 240.

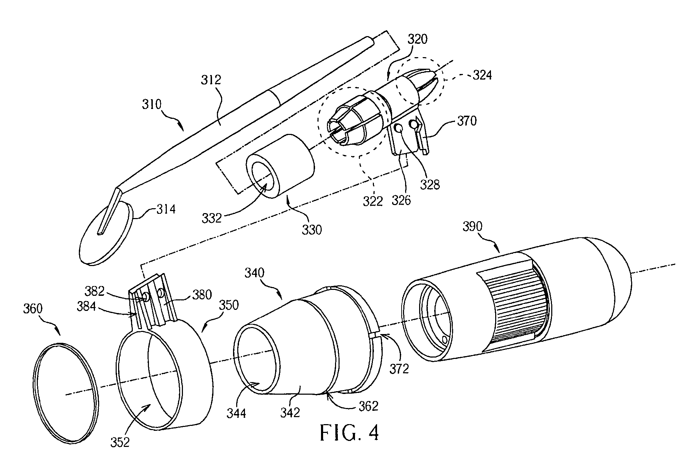

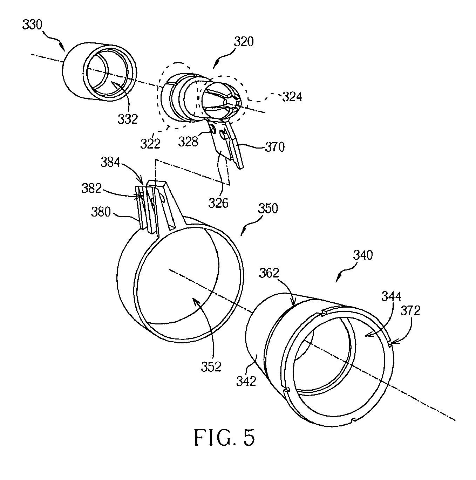

Please refer now to FIG. 4 and FIG. 5. FIG. 4 is an exploded view of the reflecting-type optical inspection apparatus 210, and FIG. 5 is a partial exploded view of the reflecting-type optical inspection apparatus 210. As shown, the holding component 320 includes a first clamping device 322, a second clamping device 324, a first connector 326, and an elongate protrusion 370. In this embodiment, the first clamping device 322 is implemented with a flexible grip for holding an elongate handle 312 of the dental mirror 310. Since the first clamping device 322 is flexible, the dental mirror 310 can detach from the first clamping device 322. The second clamping device 324 in this embodiment is also implemented with a flexible grip for holding the elongate handle 312 of the dental mirror 310, so that the holding component 320 can hold the dental mirror 310 in a more stable way.

The second clamping device 324 may be omitted in some embodiments where the first clamping device 322 alone is able to stably hold the dental mirror 310. In addition, the first and second clamping devices 322 and 324 can be replaced by a flexible sleeve where the elongate handle 312 can be inserted thereinto (or pass therethrough) and held.

The first connector 326 extends outward from the holding component 320 and has a plurality of projections 328 defined thereon. The first connector 326 connects the holding component 320 with the outer portion 350.

The fastener 330 of this embodiment is implemented with a sleeve having a through hole 332 to which the first clamping device 322 of the holding component 320 can be inserted and secured therein. When the first clamping device 322 is inserted into the through hole 332 of the fastener 330, the inner surface of the fastener 330 contacts with and presses on the first clamping device 322, so that the holding component 320 can hold the dental mirror 310 more securely. Instead of a sleeve, the fastener 330 can also be implemented with a clamping device, a hoop, or any other component that can enhance the holding function of the first clamping device 322 when engaged with the holding component 320. Additionally, the fastener 330 may be omitted in some embodiments where the holding component 320 alone is able to firmly hold the dental mirror 310.

In accordance with the foregoing, the holding component 320 may function alone or cooperate with the fastener 330 to serve as a holding portion of the reflecting-type optical inspection apparatus 210.

As shown in FIG. 4 and FIG. 5, the inner portion 340 of this embodiment includes a light mask 342, a hollow portion 344, an annular groove 362, and a plurality of cavities 372. The light mask 342 is formed at one end of the inner portion 340. The hollow portion 344 is a through hole defined longitudinally through the inner portion 340, and the handheld digital image sensing module 390 is detachably disposed in the hollow portion 344 of the inner portion 340. The annular groove 362 is formed on the surface of the inner portion 340. The cavities 372 are defined on an annular outer flange extending radially outward from one end of the inner portion 340.

The outer portion 350 has a base portion having a hollow portion 352 and a second connector 380 formed on the base portion. As shown, the base portion of the outer portion 350 may be made in the form of a sleeve, and the hollow portion 352 may be a rounded through hole defined through the base portion of the outer portion 350. In this embodiment, the second connector 380 is made in the form of two parallel plates projecting from the base portion of the outer portion 350 and has a slot 384 formed by a gap between the two plates. Each plate of the second connector 380 has a plurality of openings 382 defined thereon. When the first connector 326 of the holding component 320 is inserted into the slot 384 of the second connector 380, each of the projections 328 on the first connector 326 engages with a corresponding opening 382, so that the holding component 320 can be connected and secured with the outer portion 350.

When the inner portion 340 is inserted into the hollow portion 352 of the outer portion 350, the outer portion 350 can rotate with respect to the inner portion 340, since the hollow portion 352 of the outer portion 350 is a rounded through hole. Thus, the outer portion 350 is rotatably engaged with the inner portion 340, and the second connector 380 on the outer portion 350 is rotatable with respect to the handheld digital image sensing module 390. In addition, the holding component 320 is also rotatable with respect to the handheld digital image sensing module 390 if connected with the outer portion 350 through the engagement between the first connector 326 and the second connector 380.

In the above descriptions, the connection between the holding component 320 and the outer portion 350 is detachable. This is achieved by the cooperation of separate parts (i.e., first connector 326 and the second connector 380). However, this is simply an exemplary embodiment and should not be interpreted to restrict the present invention in any way. The holding component 320 and the outer portion 350 may be configured to be fixedly connected together and not detachable. For example, the holding component 320 and the outer portion 350 can be integrally formed.

The limiting structure 360 of this embodiment is implemented with a mounting collar. After the inner portion 340 is inserted into the hollow portion 352 of the outer portion 350, the limiting structure 360 is placed around the annular groove 362 of the inner portion 340 and attaches to the outer portion 350 to further secure the outer portion 350 onto the inner portion 340. Instead of a mounting collar, the limiting structure 360 can be made in the form of a hoop, a sleeve, or any other component that can achieve the same purpose.

In addition, the limiting structure 360 may be fixedly attached to the base portion of the outer portion 350 or formed on the base portion of the outer portion 350. In other embodiments, the limiting structure 360 may be fixedly attached to the annular groove 362 of the inner portion 340 or formed on the surface of the inner portion 340 to replace the annular groove 362. Moreover, the limiting structure 360 may be omitted in some embodiments where the inner portion 340 and/or the outer portion 350 is dimensioned such that the outer portion 350 can be secured onto the inner portion 340 without the assistance of the limiting structure 360.

In the foregoing descriptions, the handheld digital image sensing module 390 is detachably disposed in the hollow portion 344 of the inner portion 340. This is merely an exemplary embodiment and should not be interpreted to restrict or limit the present invention in any way. For example, the handheld digital image sensing module 390 can, in another embodiment, be detachably disposed in the hollow portion 352 of the outer portion 350 and directly engaged with the outer portion 350 without using the inner portion 340 as an intermediary between the outer portion 350 and the handheld digital image sensing module 390.

In accordance with the foregoing, the outer portion 350 may function alone or cooperate with the inner portion 340 to serve as a body; and such body may function alone or cooperate with the limiting structure 360 to serve as a connecting portion of the reflecting-type optical inspection apparatus 210.

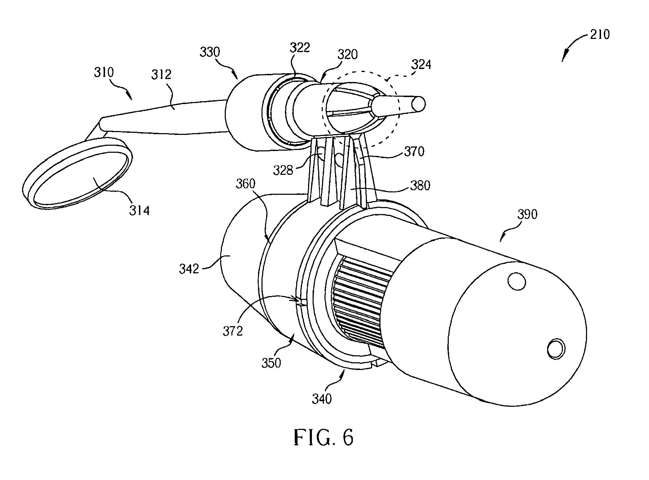

Please refer now to FIG. 6, which is another perspective view of the reflecting-type optical inspection apparatus 210, as well as FIG. 4 and FIG. 5. As shown, when the first connector 326 of the holding component 320 is inserted into the slot 384 of the second connector 380 on the outer portion 350, a free end of the protrusion 370 engages with one of the plurality of cavities 372 formed on the annular outer flange of the inner portion 340. The engagement of the free end of the protrusion 370 and the cavity 372 on the inner portion 340 positions the holding component 320 in a first predetermined position. In this embodiment, four cavities 372 are formed and evenly distributed on the annular outer flange of the inner portion 340.

As the holding component 320 (or the second connector 380) rotates with respect to the handheld digital image sensing module 390, the protrusion 370 bends (e.g., the sufficiently malleable protrusion could flex or arcuate) so that its free end disengages from the original cavity 372. When the holding component 320 (or the second connector 380) rotates 90 degrees with respect to the handheld digital image sensing module 390, the free end of the protrusion 370 of the holding component 320 would engage with the next cavity 372 on the inner portion 340 and thus position the holding component 320 in a second predetermined position.

As can be inferred from the above descriptions, the protrusion 370 on the holding component 320 and the cavities 372 on the inner portion 340 serve as positioning devices for guiding the rotation angle of the holding component 320 (or the second connector 380). In some embodiments, the annular outer flange of the inner portion 340 is omitted and the cavities 372 are instead formed on the handheld digital image sensing module 390.

Since the holding component 320 is rotatable with respect to the handheld digital image sensing module 390, the user is allowed to adjust the observation angle of the dental mirror 310 by rotating the holding component 320 or the outer portion 350 while maintaining the handheld digital image sensing module 390 in the same viewing angle. As a result, a diagnostician could, e.g., rotate the holding component 320 as needed so that the image shown on the display 240 would more or less maintain the same orientation.

Additionally, the dental mirror 310 could be a general dental mirror, so the reflective surface 314 can be easily inserted into any position within the mouth cavity, thereby reducing the visually-inaccessible areas of the mouth cavity. Another advantage is that because the dental mirror 310 is detachable from the reflecting-type optical inspection apparatus 210, the disinfection or replacement of a used dental mirror is very convenient, and would not result in hygienic concerns.

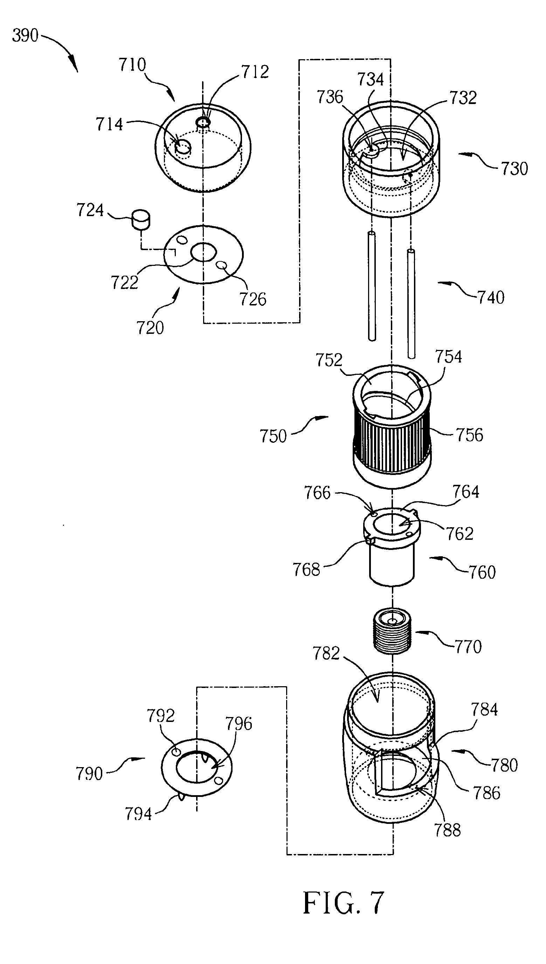

Please refer to FIG. 7, which illustrates an exploded view of the handheld digital image sensing module 390 according to an exemplary embodiment. The handheld digital image sensing module 390 comprises a back cover 710, a photo sensor assembly 720, a cap 730, at least one guide (two are shown in this embodiment) 740, a telescopic tube assembly, a casing 780, and a light module 790, wherein the telescopic tube assembly of this embodiment comprises an outer tube 750, an inner tube 760, and a lens 770.

The back cover 710 comprises two through holes 712 and 714. In the embodiments where the connection 230 between the reflecting-type optical inspection apparatus 210 and the computer 220 is a physical cable, the connection 230 is connected with the photo sensor assembly 720 via the through hole 712 situated on the back cover 710.

The photo sensor assembly 720 has a circuit board, an optical sensor 722 mounted to the circuit board, a control interface 724 electrically coupled with the circuit board, and at least one electrode (two are shown in this embodiment) 726 formed on the circuit board. When operating, the optical sensor 722 is arranged facing to the lens 770 and senses images from the light passing through the lens 770. In one embodiment, the control interface 724 is a camera shutter button disposed on the surface of the handheld digital image sensing module 390 via the through hole 714 situated on the back cover 710. When a user presses the control interface 724, the control interface 724 triggers a control signal. In response to the control signal, the photo sensor assembly 720 captures and saves the currently sensed image (i.e., the image currently shown on the display 240) as an image file. This function facilitates the user in establish the inspection records (e.g., a case history). The control interface 724 may instead be implemented with a touch panel, a voice receiving device, or any other device that can trigger a control signal in responsible to the user's operation (e.g., a finger knock, a vocal command, etc.).

The cap 730 is tubular and attached to the back cover 710 and the casing 780. The cap 730 includes a hollow portion 732, an inner surface, and at least one protrusion (two are shown in this embodiment) 734. The photo sensor assembly 720 is disposed in the hollow portion 732. The protrusions 734 extend radially inward from the inner surface of the cap 730 and each protrusion 734 includes a limiting structure 736.

The casing 780 is tubular and includes a through hole 782, an inner surface, an opening 784, and an annular inner flange 786. The through hole 782 is defined longitudinally through the casing 780. The opening 784 is defined on a side of the casing 780. The annular inner flange 786 extends radially inward from the inner surface of the casing 780 and includes at least one limiting structure (two are shown in this embodiment) 788.

The telescopic tube assembly is disposed within the casing 780. As mentioned previously, the telescopic tube assembly of this embodiment includes the outer tube 750, the inner tube 760, and the lens 770. The outer tube 750 is tubular and disposed rotatably in the through hole 782 of the casing 780. The outer tube 750 includes an inner surface 752, at least one spiral track (two are shown in the embodiment) 754, and an annular knob 756. The spiral tracks 754 are formed on the inner surface 752 of the outer tube 750. The annular knob 756 is formed on the outer surface of the outer tube 750 and can be viewed through the opening 784 of the casing 780 when the outer tube 750 is disposed in the through hole 782 of the casing 780.

The inner tube 760 is disposed movably within the outer tube 750 and includes a through hole 762, an annular outer flange 764, at least one engaging element (two are shown in this embodiment) 766, and at least one guiding element (two are shown in this embodiment) 768. The through hole 762 is defined longitudinally through the inner tube 760, and the lens 770 is attached to the through hole 762. The annular outer flange 764 extends radially outward from one end of the inner tube 760 and has an outer edge.

The two guiding elements 768 are movably joined with the two spiral tracks 754 of the outer tube 750, respectively. The implementation of each guiding element 768 may vary with the implementation of the corresponding spiral track 754. For example, in one embodiment where the spiral track 754 is implemented with a spiral groove, the corresponding guiding element 768 may be implemented with a tab protruding from the inner tube 760 (or formed on the outer edge of the annular outer flange 764 of the inner tube 760). In another embodiment where the spiral track 754 is implemented with a spiral protruding stripe, the corresponding guiding element 768 may be implemented with a matching notch formed on the inner tube 760 (or formed on the outer edge of the annular outer flange 764 of the inner tube 760).

The light module 790 is disposed at one end of the casing 780 and includes at least one electrode (two are shown in this embodiment) 792, at least one light source (e.g., LED) 794, and a through hole 796. In conventional dental field, a dental lamp is commonly used in combination with a dental mirror to assist viewing while inspecting the oral tissue. The conventional dental lamp provides a large beam of light which may be focused in the general area of a patient's mouth. The diagnostician's hands and instruments often shade the area which is being viewed, thereby requiring the diagnostician to keep adjusting his/her posture so as not to shade the area being viewed. During an oral inspection or intra-oral operation, the conventional dental lamp must constantly be adjusted, thereby resulting in considerable time loss and frustration for the diagnostician. The light module 790 can be employed alone or to cooperate with other light source to provide sufficient illumination for intra-oral inspection and operation, and the above problem can therefore be resolved.

The two guides 740 are slidably engaged with the inner tube 760 and disposed between the casing 780 and the cap 730. Each of the two guides 740 has two ends; the first end corresponds to one of the two limiting structures 736 of the cap 730 while the second end corresponds to one of the two limiting structures 788 of the casing 780. The two guides 740 may be implemented with two guiding rods.

In one embodiment, the two limiting structures 736 of the cap 730 are implemented with two mounting holes defined through the protrusion 734, the two limiting structures 788 of the casing 780 are implemented with two mounting holes defined through the annular inner flange 786, and the two engaging elements 766 of the inner tube 760 are implemented with two through holes defined through the annular outer flange 764. In this case, the first end of each guide 740 passes through a corresponding mounting hole (serving as the limiting structure 736) of the cap 730 and contacted with a corresponding electrode 726 on the photo sensor assembly 720, while the second end of the guide 740 passes through a corresponding through hole (serving as the engaging element 766) on the annular outer flange 764 of the inner tube 760 and a corresponding mounting hole (serving as the limiting structure 788) of the casing 780, and then contacted with a corresponding electrode 792 on the light module 790.

The guides 740 can be made of conductive materials so that the electricity to the light source 794 of the light module 790 can be conducted via the guides 740.

In the above descriptions, the limiting structures 736, the limiting structures 788, and the engaging elements 766 are implemented with mounting holes or through holes. This is merely exemplary and should not be interpreted to restrict or limit the invention in any way. For example, each limiting structure 736 of the cap 730 and each limiting structure 788 of the casing 780 may instead be implemented with a notch or any other structure that is able to limit the transverse movement of a corresponding guide 740. In addition, each engaging element 766 of the inner tube 760 may instead be implemented with a C-shaped notch or any other structure that can slidably engage with a corresponding guide 740.

Because the two guides 740 are slidably engaged with the two engaging elements 766 of the inner tube 760, rotating the annular knob 756 of the outer tube 750 would cause the inner tube 760 to slide along the guides 740 in the outer tube 750. This is due to the relative movement between the spiral tracks 754 of the outer tube 750 and the guiding elements 768 of the inner tube 760. That is, the inner tube 760 can move longitudinally in the outer tube 750 when the user rotates the outer tube 750 by turning the annular knob 756.

As a result, the magnification of the handheld digital image sensing module 390 can change with the variation of a distance between the optical sensor 722 and the lens 770. Moreover, the focal length of the handheld digital image sensing module 390 can change with the variation of a distance between the lens 770 and the target object 302 aimed by the handheld digital image sensing module 390.

The magnification and focal length of the handheld digital image sensing module 390 can be changed by adjusting the telescopic tube assembly without replacing the lens 770 or moving the target object 302 aimed by the handheld digital image sensing module 390. Therefore, the operation of the handheld digital image sensing module 390 is convenient and efficient.

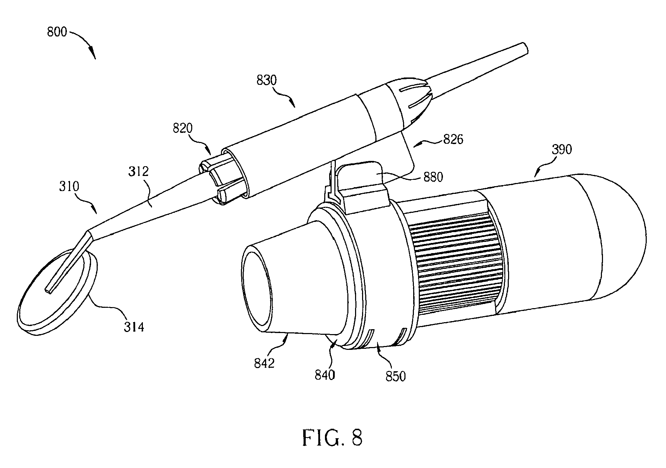

Please refer now to FIG. 8, which illustrates a perspective view of a reflecting-type optical inspection apparatus 800 according to a second exemplary embodiment. The reflecting-type optical inspection apparatus 800 comprises a holding component 820, a first connector 826, a fastener 830, an inner portion 840, a light mask 842, an outer portion 850, and the handheld digital image sensing module 390. The holding component 820 is configured for detachably holding the external device (e.g., a dental mirror) 310 having a reflective surface 314. The fastener 830 is detachably engaged with the holding component 820. The outer portion 850 is rotatably engaged with the inner portion 840 and detachably engaged with the holding component 820. The handheld digital image sensing module 390 is detachably engaged with the inner portion 840. Different from the embodiment shown in FIG. 3 ∼ FIG. 6, where the first connector 326 is attached to the holding component 320, the first connector 826 of this embodiment is detachably attached to the fastener 830.

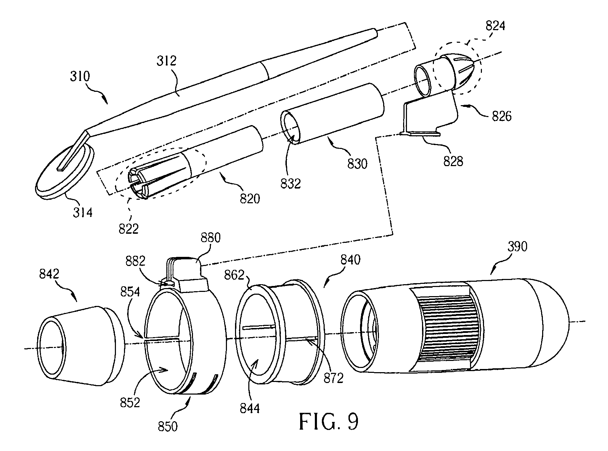

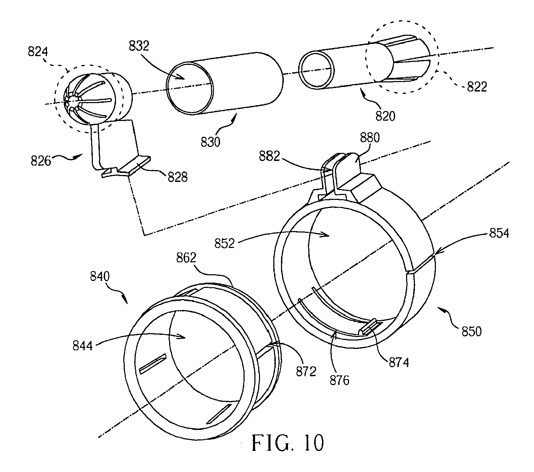

Referring to FIG. 9 and FIG. 10. FIG. 9 is an exploded view of the reflecting-type optical inspection apparatus 800, and FIG. 10 is a partial exploded view of the reflecting-type optical inspection apparatus 800. As shown, the holding component 820 includes a first clamping device 822. In this embodiment, the first clamping device 822 is implemented with a flexible grip for holding an elongate handle 312 of the dental mirror 310. Since the first clamping device 822 is flexible, the dental mirror 310 can detach from the first clamping device 822.

The first connector 826 includes a second clamping device 824 and a T-shaped plug 828. The T-shaped plug 828 is employed to connect the first connector 826 with the outer portion 850. The second clamping device 824 in this embodiment is also implemented with a flexible grip for holding the elongate handle 312 of the dental mirror 310, so that the dental mirror 310 can be held in a more stable way when the holding component 820 cooperates with the first connector 826 through the connection of the fastener 830. The second clamping device 824 may be omitted in some embodiments where the first clamping device 822 alone is able to stably hold the dental mirror 310. In addition, each of the first and second clamping devices 822 and 824 can be replaced by a flexible sleeve where the elongate handle 312 can be inserted thereinto (or pass therethrough) and held.

The fastener 830 of this embodiment is implemented with a sleeve having a through hole 832 to which the holding component 820 can be inserted and secured therein. When the first clamping device 822 is inserted into the through hole 832 of the fastener 830, the inner surface of the fastener 830 contacts with and presses on the first clamping device 822, so that the holding component 820 can hold the dental mirror 310 more securely. Instead of a sleeve, the fastener 830 can also be implemented with a clamping device, a hoop, or any other component that can enhance the holding function of the first clamping device 822 when engaged with the holding component 820. Additionally, the fastener 830 may be omitted in some embodiments where the holding component 820 alone is able to firmly hold the dental mirror 310.

From one aspect of the foregoing embodiments, the holding component 820 may cooperate with the fastener 830 and the first connector 826 to serve as a holding portion of the reflecting-type optical inspection apparatus 800.

As shown in FIG. 9 and FIG. 10, the inner portion 840 of this embodiment includes a hollow portion 844, a limiting structure 862, and a plurality of openings 872. The light mask 842 is detachably attached to one end of the inner portion 840. The hollow portion 844 is a through hole defined longitudinally through the inner portion 840, and the handheld digital image sensing module 390 is detachably disposed in the hollow portion 844 of the inner portion 840. The limiting structure 862 is formed on the surface of the inner portion 840. The openings 872 are defined on the surface of the inner potion 340.

The outer portion 850 has a base portion having a hollow portion 852 and a break 854, and a second connector 880 formed on the base portion. The outer portion 850 further comprises a flexible arm formed by a protrusion 874 and a U-shaped break 876 defined on the base portion. As shown, the base portion of the outer portion 850 may be made in the form of a sleeve having a C-shaped section. The hollow portion 852 may be a rounded through hole defined through the base portion of the outer portion 850. In this embodiment, the second connector 880 is made in the form of two parallel bended plates projecting from the base portion of the outer portion 850 and includes a T-shaped slot 882 formed by a gap between the two bended plates. When the first connector 826 of the holding component 820 engages with the second connector 880, the T-shaped plug 828 on the first connector 826 is inserted into the T-shaped slot 882 of the second connector 880, so that the first connector 826 can be connected and secured with the outer portion 850.

When the inner portion 840 is inserted into the hollow portion 852 of the outer portion 850, the outer portion 850 can be rotated with respect to the inner portion 840 since the hollow portion 852 of the outer portion 850 is a rounded through hole. Thus, the outer portion 850 is rotatably engaged with the inner portion 840, and the second connector 880 on the outer portion 850 and the first connector 826 engaged with the second connector 880 are rotatable with respect to the handheld digital image sensing module 390. In addition, the fastener 830 and the holding component 820 are also rotatable with respect to the handheld digital image sensing module 390 if attached to the first connector 826.

In the above descriptions, the connection between the first connector 826 and the outer portion 850 is detachable and achieved by the cooperation of separate parts (i.e., the T-shaped plug 828 and the T-shaped slot 882). This is merely an exemplary embodiment and should not be interpreted to restrict or limit the present invention in any way. The first connector 826 and the outer portion 850 may be configured to be fixedly connected together and not detachable. For example, the first connector 826 and the outer portion 850 can be integrally formed.

The limiting structure 862 of this embodiment is implemented with an annular outer flange formed on the surface of the inner portion 840. When the inner portion 840 is inserted into the hollow portion 852 of the outer portion 850, the base portion of the outer portion 850 slightly deforms to allow the limiting structure 862 to pass through. After passing the hollow portion 852, the limiting structure 862 attaches with the outer portion 850 to further secure the outer portion 850 onto the inner portion 840. Instead of an annular outer flange, the limiting structure 862 can be made in the form of one or more projections, or any other component that can achieve the same purpose.

The limiting structure 862 may be omitted in some embodiments where the inner portion 840 and/or the outer portion 850 is dimensioned such that the outer portion 850 can be secured onto the inner portion 840 without the assistance of the limiting structure 862.

In the foregoing descriptions, the handheld digital image sensing module 390 is detachably disposed in the hollow portion 844 of the inner portion 840. The handheld digital image sensing module 390 can instead be detachably disposed in the hollow portion 852 of the outer portion 850 and directly engaged with the outer portion 850 without using the inner portion 840 as an intermediary between the outer portion 850 and the handheld digital image sensing module 390.

From one aspect of the foregoing embodiments, the outer portion 850 may function alone or cooperate with the inner portion 840 to serve as a body, and such body may function alone or cooperate with the limiting structure 862 to serve as a connecting portion of the reflecting-type optical inspection apparatus 800.

As shown on FIG. 9 and FIG. 10, when the inner portion 840 is inserted into the outer portion 850, the protrusion 874 of the flexible arm of the outer portion 850 engages with one of the openings 872 on the inner portion 840. The engagement of the protrusion 874 and the opening 872 positions the second connector 880 (and the first connector 826 and associated holding component 820) in a first predetermined position. In this embodiment, four openings 872 are formed and evenly distributed on the surface of the inner portion 840.

As the holding component 820 (or the second connector 880) rotates with respect to the handheld digital image sensing module 390, the flexible arm of the outer portion 850 deforms so that the protrusion 874 disengages from the original opening 872. When the holding component 820 (or the second connector 880) rotates 90 degrees with respect to the handheld digital image sensing module 390, the protrusion 874 of the outer portion 850 would engage with the next opening 872 on the inner portion 840 and thus position the holding component 820 in a second predetermined position.

As can be inferred from the above descriptions, the protrusion 874 on the outer portion 850 and the openings 872 on the inner portion 840 serve as positioning devices for guiding the rotation angle of the holding component 820 (or the second connector 880). In some embodiments where the inner portion 840 is omitted, the openings 872 may instead be formed on the handheld digital image sensing module 390.

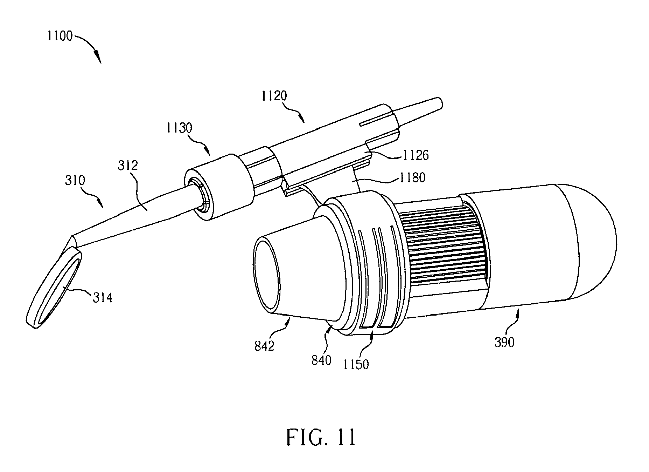

Please refer to FIG. 11, which illustrates a perspective view of a reflecting-type optical inspection apparatus 1100 according to a third exemplary embodiment. The reflecting-type optical inspection apparatus 1100 comprises a holding component 1120, a fastener 1130, the inner portion 840, the light mask 842, an outer portion 1150, and the handheld digital image sensing module 390. The holding component 1120 is configured for detachably holding the external device (e.g., a dental mirror) 310 having a reflective surface 314. The fastener 1130 is detachably engaged with the holding component 1120. The outer portion 1150 is rotatably engaged with the inner portion 840 and detachably engaged with the holding component 1120. The handheld digital image sensing module 390 is detachably engaged with the inner portion 840.

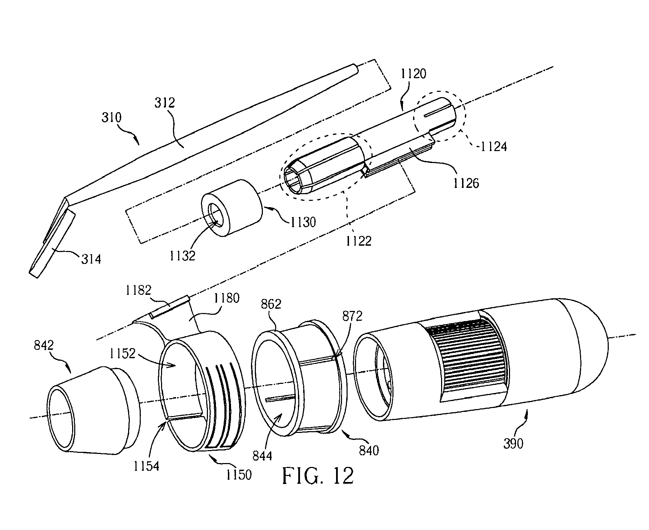

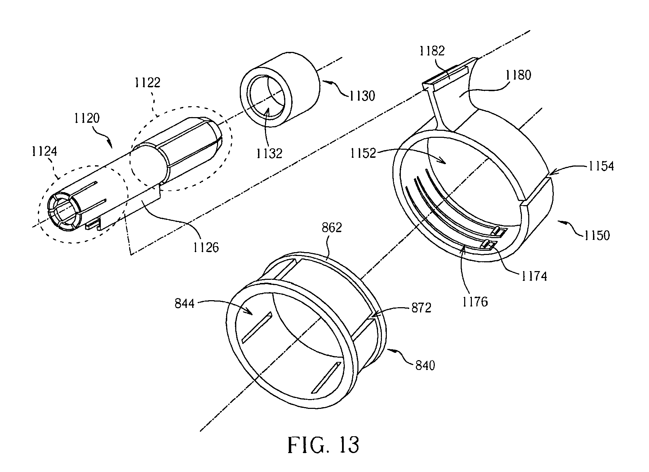

Referring to FIG. 12 and FIG. 13. FIG. 12 is an exploded view of the reflecting-type optical inspection apparatus 1100, and FIG. 13 is a partial exploded view of the reflecting-type optical inspection apparatus 1100. As shown, the holding component 1120 includes a first clamping device 1122, a second clamping device 1124, and a first connector 1126. In this embodiment, the first clamping device 1122 is implemented with a flexible grip for holding the elongate handle 312 of the dental mirror 310. Since the first clamping device 322 is flexible, the dental mirror 310 can detach from the first clamping device 322. The second clamping device 1124 in this embodiment is also implemented with a flexible grip for holding the elongate handle 312 of the dental mirror 310, so that the holding component 1120 can hold the dental mirror 310 in a more stable way.

The second clamping device 1124 may be omitted in some embodiments where the first clamping device 1122 alone is able to stably hold the dental mirror 310. In addition, the first and second clamping devices 1122 and 1124 can be replaced by a flexible sleeve where the elongate handle 312 can be inserted thereinto (or pass therethrough) and held.

The first connector 1126 extends outward from the holding component 1120 and includes a slot defined therein. The first connector 1126 is employed to connect the holding component 1120 with the outer portion 1150. Different from the afore-mentioned reflecting-type optical inspection apparatus 800 where the first connector 826 is detachably attached to the fastener 830, the first connector 1126 of this embodiment is attached to the holding component 1120.

The fastener 1130 of this embodiment is implemented with a sleeve having a through hole 1132 to which the first clamping device 1122 of the holding component 1120 can be inserted and secured therein. When the first clamping device 1122 is inserted into the through hole 1132 of the fastener 1130, the inner surface of the fastener 1130 contacts with and presses on the first clamping device 1122, so that the holding component 1120 can hold the dental mirror 310 more securely. Instead of a sleeve, the fastener 1130 can also be implemented with a clamping device, a hoop, or any other component that can enhance the holding function of the first clamping device 1122 when engaged with the holding component 1120. Additionally, the fastener 1130 may be omitted in some embodiments where the holding component 1120 alone is able to firmly hold the dental mirror 310.

In accordance with the foregoing, the holding component 1120 may function alone or cooperate with the fastener 1130 to serve as a holding portion of the reflecting-type optical inspection apparatus 1100.

As shown in FIG. 12 and FIG. 13, the inner portion 840 in the reflecting-type optical inspection apparatus 1100 is the same as that in the afore-mentioned reflecting-type optical inspection apparatus 800. And thus, the descriptions relating to inner portion 840 will not be repeated here.

The outer portion 1150 has a base portion having a hollow portion 1152 and a break 1154, and a second connector 1180 formed on the base portion. The outer portion 1150 further comprises two flexible arms each having a protrusion 1174 and a U-shaped break 1176 defined on the base portion. As shown, the base portion of the outer portion 1150 may be made in the form of a sleeve having a C-shaped section. The hollow portion 1152 may be a rounded through hole defined through the base portion of the outer portion 1150. In this embodiment, the second connector 1180 extends outward from the base portion of the outer portion 1150 and includes two projections 1182 defined thereon.

When the first connector 1126 of the holding component 1120 engages with the second connector 1180, the projections 1182 on the second connector 1180 are inserted into the slot of the first connector 1126, so that the holding component 1120 can be connected and secured with the outer portion 1150.

When the inner portion 840 is inserted into the hollow portion 1152 of the outer portion 1150, the outer portion 1150 can be rotated with respect to the inner portion 840 since the hollow portion 1152 of the outer portion 1150 is a rounded through hole. Thus, the outer portion 1150 is rotatably engaged with the inner portion 840, and the second connector 1180 on the outer portion 1150 is rotatable with respect to the handheld digital image sensing module 390. In addition, the holding component 1120 is also rotatable with respect to the handheld digital image sensing module 390 if the holding component 1120 is connected with the outer portion 1150 through the engagement between the first connector 1126 and the second connector 1180.

In the above descriptions, the connection between the first connector 826 and the outer portion 1150 is detachable and achieved by the cooperation of separate parts (i.e., first connector 1126 and the second connector 1180). In other embodiments, the first connector 1126 and the outer portion 1150 may be configured to be fixedly connected together and not detachable. For example, the holding component 1120 and the outer portion 1150 can be integrally formed.

As described previously, the limiting structure 862 of this embodiment is implemented with an annular outer flange formed on the surface of the inner portion 840. When the inner portion 840 is inserted into the hollow portion 1152 of the outer portion 1150, the base portion of the outer portion 1150 slightly deforms to allow the limiting structure 862 to pass through. After passing the hollow portion 1152, the limiting structure 862 contacts with the outer portion 1150 to further secure the outer portion 1150 onto the inner portion 840.

The limiting structure 862 may be omitted in some embodiments where the inner portion 840 and/or the outer portion 1150 is dimensioned such that the outer portion 1150 can be secured onto the inner portion 840 without the assistance of the limiting structure 862.

Similar to the previous embodiments, the handheld digital image sensing module 390 can instead be detachably disposed in the hollow portion 1152 of the outer portion 1150 and directly engaged with the outer portion 1150 without using the inner portion 840 as an intermediary between the outer portion 1150 and the handheld digital image sensing module 390.

From one aspect of the foregoing embodiments, the outer portion 1150 may function alone or cooperate with the inner portion 840 to serve as a body, and such body may function alone or cooperate with the limiting structure 862 to serve as a connecting portion of the reflecting-type optical inspection apparatus 1100.

As shown in FIG. 12 and FIG. 13, when the inner portion 840 is inserted into the outer portion 1150, the protrusion 1174 of the flexible arm of the outer portion 1150 engages with one of the openings 872 on the inner portion 840. The engagement of the protrusion 1174 and the opening 872 positions the second connector 1180 (and associated holding component 1120) in a first predetermined position. In this embodiment, four openings 872 are formed and evenly distributed on the surface of the inner portion 840.

As the holding component 1120 (or the second connector 1180) rotates with respect to the handheld digital image sensing module 390, the flexible arm of the outer portion 1150 deforms so that the protrusion 1174 disengages from the original opening 872. When the holding component 1120 (or the second connector 1180) rotates 90 degrees with respect to the handheld digital image sensing module 390, the protrusion 1174 of the outer portion 1150 would engage with the next opening 872 on the inner portion 840 and thus position the holding component 1120 in a second predetermined position.

As can be inferred from the above descriptions, the protrusion 1174 on the outer portion 1150 and the openings 872 on the inner portion 840 serve as positioning devices for guiding the rotation angle of the holding component 1120 (or the second connector 1180).

The invention can be summarized as a reflecting-type optical inspection apparatus includes: a holding portion configured for detachably holding an external device having a reflective surface; a connecting portion engaged with the holding portion; and a digital image sensing module engaged with the connecting portion for sensing light reflected from the reflective surface of the external device.

## Claims

1. A reflecting-type optical inspection apparatus comprising:
a holding portion comprising:
a first connector; and
a holding component configured for detachably holding an external device having a reflective surface;
a connecting portion comprising:
a body having a hollow portion; and
a second connector formed on the body and engaged with the first connector of the holding portion; and
a digital image sensing module disposed in the hollow portion of the body for sensing light reflected from the reflective surface.

2. The reflecting-type optical inspection apparatus of claim 1, wherein the second connector of the connecting portion is detachably engaged with the first connector of the holding portion, and/or the digital image sensing module is detachably disposed in the hollow portion of the body.

3. The reflecting-type optical inspection apparatus of claim 1 or 2, wherein the digital image sensing module comprises:
a casing having a through hole defined longitudinally through the casing and an opening defined on the casing;
a cap attached to the casing and having a hollow portion;
at least one guide disposed between the casing and the cap; and
a telescopic tube assembly disposed within the casing and comprising
an outer tube rotatably disposed within the casing and having an inner surface, at least one spiral track formed on the inner surface, and a knob corresponding to the opening of the casing;
an inner tube movably disposed within the outer tube and having a through hole, at least one engaging element movably contacted with the at least one guide and at least one guiding element movably contacted with the at least one spiral track of the outer tube; and
a lens attached to the through hole of the inner tube.

4. The reflecting-type optical inspection apparatus of claim 3, wherein
the at least one engaging element of the inner tube comprises a first through hole; and
the at least one guide comprises a guiding rod passing through the first through hole.

5. The reflecting-type optical inspection apparatus of claim 3 or 4, wherein
the cap is provided with at least one first limiting structure;
the casing is provided with at least one second limiting structure; and
each guide has two ends respectively corresponding to the first limiting structure and the second limiting structure.

6. The reflecting-type optical inspection apparatus of any of claims 3 to 5, wherein the digital image sensing module further comprises:
at least one light source disposed at one end of the digital image sensing module;
wherein the electricity to the at least one light source is conducted via the at least one guide.

7. The reflecting-type optical inspection apparatus of any of claims 1 to 6, wherein the digital image sensing module further comprises:
a photo sensor assembly for processing sensed images in response to a control signal.

8. The reflecting-type optical inspection apparatus of claim 7, wherein the digital image sensing module further comprises:
a control interface disposed on the digital image sensing module and electrically coupled with the photo sensor assembly for triggering the control signal in response to a user operation.

9. The reflecting-type optical inspection apparatus of any of claims 1 to 8, wherein the holding component comprises at least a flexible sleeve or a clamping device.

10. The reflecting-type optical inspection apparatus of any of claims 1 to 9, wherein the holding portion further comprises:
a fastener detachably engaged with the holding component;
wherein the first connector is attached to the fastener or the holding component.

11. The reflecting-type optical inspection apparatus of claim 10, wherein the fastener comprises a sleeve, a clamping device, or a hoop.

12. The reflecting-type optical inspection apparatus of any of claims 1 to 11, wherein the second connector of the connecting portion is configured rotatable with respect to the digital image sensing module.

13. The reflecting-type optical inspection apparatus of any of claims 1 to 12, wherein the body of the connecting portion further comprises:
an inner portion having the hollow portion; and
an outer portion rotatably engaged with the inner portion;
wherein the second connector of the connecting portion is formed on the outer portion.

14. The reflecting-type optical inspection apparatus of claim 13, wherein the body of the connecting portion further comprises:
at least one limiting structure contacted with the outer portion;
wherein the at least one limiting structure is fixedly attached to the inner portion or the outer portion, formed on the inner portion or the outer portion, or detachable from the inner portion and the outer portion.

15. The reflecting-type optical inspection apparatus of claim 12, comprising:
one or more positioning devices configured for guiding the rotation angle of the second connector of the connecting portion;
wherein the one or more positioning devices are disposed on at least one of the holding portion, the connecting portion, and the digital image sensing module.

16. The reflecting-type optical inspection apparatus of claim 15, wherein the one or more positioning devices comprise a cavity and/or a protrusion.

17. A reflecting-type optical inspection apparatus comprising:
a holding component configured for detachably holding an external device having a reflective surface;
a fastener detachably engaged with the holding component;
a first connector attached to the fastener or the holding component;
an inner portion having a hollow portion;
an outer portion rotatably engaged with the inner portion;
at least one limiting structure fixedly attached to the inner portion or the outer portion, formed on the inner portion or the outer portion, or detachable from the inner portion and the outer portion;
one or more positioning devices configured for positioning the holding component to a predetermined position;
a second connector formed on the outer portion and detachably engaged with the first connector; and
a digital image sensing module detachably disposed in the hollow portion of the inner portion for sensing light reflected from the reflective surface.

18. A reflecting-type optical inspection apparatus comprising:
a holding portion configured for detachably holding an external device having a reflective surface;
a connecting portion engaged with the holding portion; and
a digital image sensing module engaged with the connecting portion for sensing light reflected from the reflective surface.

19. A dental inspection system comprising:
a dental mirror having a handle and a reflective surface;
a holding portion detachably engaged with the handle of the dental mirror;
a handheld digital image sensing module for sensing light reflected from the reflective surface;
a connecting portion engaged with the holding portion and the handheld digital image sensing module; and
a display electrically coupled with the handheld digital image sensing module for displaying images sensed by the handheld digital image sensing module;
wherein the holding portion or at least a part of the connecting portion is rotatable with respect to the handheld digital image sensing module.
